# EUROPEAN PATENT APPLICATION

(11) **EP 3 901 959 A1**
(43) Date of publication of application: **27.10.2021**
(21) Application number: 19900951.5
(22) Date of filing: 09.10.2019
(51) Int. Cl.: G16H 20/00, C12Q 1/02, G01N 33/53

(54) **HEALTH RISK INFORMATION MANAGEMENT DEVICE, HEALTH RISK INFORMATION MANAGEMENT METHOD, AND PROGRAM**

(30) Priority: 21.12.2018 JP 2018239868
(71) Applicant: I Peace, Inc., Palo Alto, CA 94303 (US); FANUC CORPORATION, Oshino-mura Minamitsuru-gun Yamanashi 401-0597 (JP)
(72) Inventor: TANABE, Koji, Palo Alto, California 94303 (US); INABA, Kiyonori, Minamitsuru-gun, Yamanashi 401-0597 (JP)
(74) Representative: MacLean, Martin Robert
(86) International application number: PCT/JP2019/039818
(87) International publication number: WO 2020/129366

(57) **Abstract**

The present invention includes predicted health risk information acquisition unit which acquires information about the predicted health risks of a user, and a cultured cell production determination unit which determines whether or not to produce cultured cells derived from the user based on the information on the predicted health risks.

## Description

### Cross-Reference to Related Application

The present application is based on Japanese Patent Application No. 2018-239868, filed on Dec. 21, 2018, the entire contents of which are incorporated herein by reference.

### Technical Field

The present invention relates to a health risk information management device, a health risk information management method, and a program.

### Background Art

In recent years, expectations for regenerative medicine using iPS cells have increased. On the other hand, for example, as disclosed in Patent Document 1, there is known a technique for estimating future health risks based on genome information, biological information such as blood pressure, and behavior information such as the amount of exercise. It is desired to realize a scheme to prepare for future regenerative medicine treatment depending on such future health risks.

### Citation List

### Patent Document

Patent Document 1: Japanese Patent Application Laid-Open No. 2017 -097895

### Summary

Since the production of iPS cells is expensive, it is desired to make suggestions to a user based on accurate information about future health risks (it is good to produce what kind of iPS cells and by what age, how likely it is to reduce the risks, and the like).

Therefore, it is an object of the present invention to predict future health risks accurately by utilizing stem cells and to realize a scheme to prepare for the future health risks.

A health risk information management device according to the present invention includes:
a predicted health risk information acquisition unit which acquires information about predicted health risks of a user; and
a cultured cell production determination unit which determines whether or not to produce cultured cells derived from the user based on the information on the predicted health risks.

A health risk information management method according to the present invention includes:
a step of acquiring information about predicted health risks of a user; and
a step of determining whether or not to produce cultured cells derived from the user based on the information on the predicted health risks.

A program according to the present invention causes a computer to function as:
a predicted health risk information acquisition unit which acquires information about predicted health risks of a user; and
a cultured cell production determination unit which determines whether or not to produce cultured cells derived from the user based on the information on the predicted health risks.

### Advantageous Effects of Invention

According to the present invention, stem cells can be utilized to predict future health risks accurately and a scheme to prepare for future health risks can be realized.

### Brief Description of Drawings

Fig. 1 is a block diagram illustrating the configuration of a health risk information management device 10 according to an embodiment of the present invention.
Fig. 2 is a block diagram illustrating functional modules implemented by a control device 11 of the health risk information management device 10 according to the embodiment of the present invention.
Fig. 3 is a flowchart of proposal processing for somatic cell production based on user's health risk information performed by the health risk information management device 10 according to the embodiment of the present invention.
Fig. 4 is a flowchart of demonstration experiments on health risks using somatic cells produced through iPS cells and proposal processing for banking of iPS cells based on the results of the demonstration experiments according to the embodiment of the present invention.
Fig. 5 is a flowchart of search processing for HLA-homozygous donors according to the embodiment of the present invention.

### Description of Embodiments

Next, a mode for carrying out the present invention will be described with reference to the accompanying drawings.

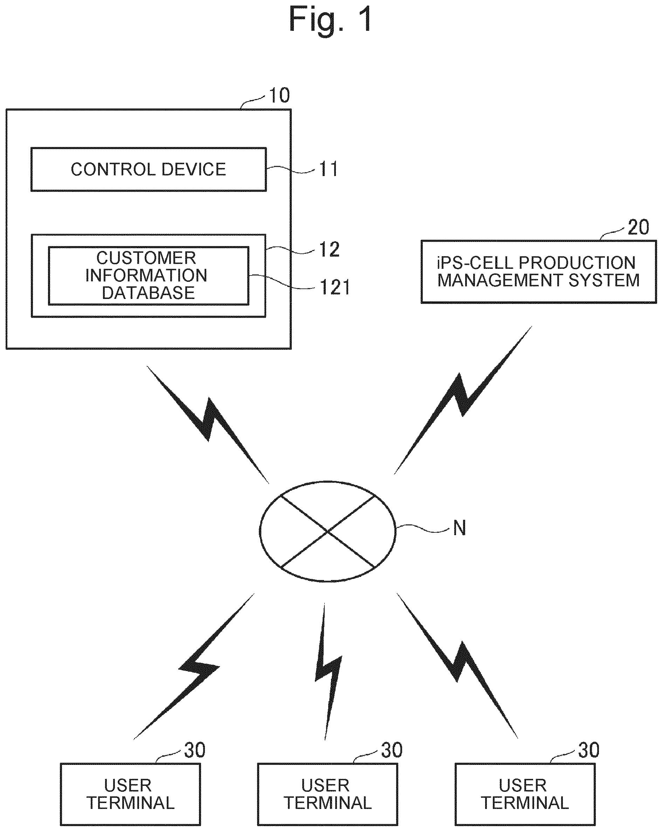

Fig. 1 is a block diagram illustrating the configuration of a health risk information management device 10 according to an embodiment of the present invention. As illustrated in Fig. 1, the health risk information management device 10 includes a control device 11 and an external storage device 12.

The control device 11 includes, as hardware, a CPU, memories such as a ROM and a RAM, an input interface, an output interface, a communication interface, and the like. The control device 11 implements various functions by the CPU executing programs stored in the ROM and the like. The external storage device 12 is a hard disk drive or the like. A customer information database 121 is implemented in the external storage device 12.

The health risk information management device 10 does not have to be configured by one computer, and it may consist of two or more computers and external storage devices distributed on a communication network. Further, as illustrated in Fig. 1, the health risk information management device 10 is connected to a cell production management system 20 and user terminals 30 through a communication line N.

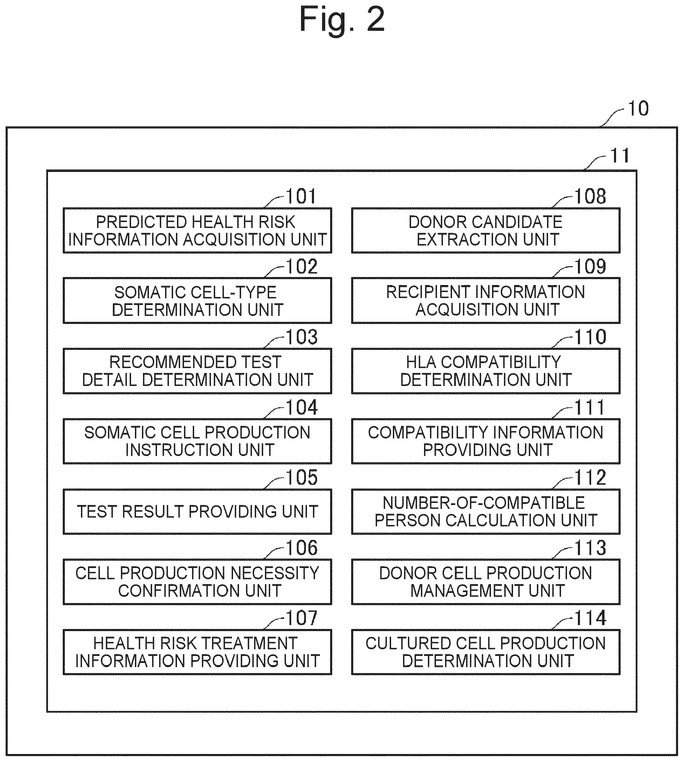

Fig. 2 is a block diagram illustrating functional modules implemented by the control device 11 of the health risk information management device 10. The functional modules include a predicted health risk information acquisition unit 101, a somatic cell-type determination unit 102, a recommended test detail determination unit 103, a somatic cell production instruction unit 104, a test result providing unit 105, a cell production necessity confirmation unit 106, a health risk treatment information providing unit 107, a donor candidate extraction unit 108, a recipient information acquisition unit 109, an HLA compatibility determination unit 110, a compatibility information providing unit 111, a number-of-compatible person calculation unit 112, a donor cell production management unit 113, and a cultured cell production determination unit 114.

In the customer information database 121, information on each user who has registered, in the health risk information management device 10, user's health risk information and information on banking of iPS cells is registered. Specifically, for example, in addition to a user ID (user identification information), the name, gender, and date of birth of the user, contact information (an email address of the user terminal 30, a phone number, or the like), and the like, information about genetic testing and health risks (possible diseases, the probability of developing each disease, when the onset of the disease is expected, HLA type, and the like), information about preserved iPS cells and somatic cells (the identification number, the type of somatic cells, the number of cells preserved, the production date, etc.), and the like are included.

The cell production management system 20 manages the production of iPS cells derived from somatic cells of the user (for example, autologous cells; cells for an autologous transplant). It is preferred that the cell production management system 20 should be a dedicated device capable of creating iPS cells, but the present invention is not limited thereto, and systems to manage the production of iPS cells in medical institutions, research institutions, and other production institutions, where iPS cells can be produced, are included. The dedicated device capable of creating iPS cells is a device capable of performing, in a closed space without human intervention, at least one or more, preferably more than two, of the following: the isolation of somatic cells (such as the isolation of peripheral blood mononuclear cells from blood or the isolation of fibroblasts from a skin piece) and the expanded culture of the isolated cells in addition to the induction, culture, preservation, and recording of iPS cells. It is also desired that the isolation of somatic cells of the user as the source of cell production (such as the isolation of peripheral blood mononuclear cells from blood or the isolation of fibroblasts from a skin piece), the expanded culture of the isolated cells, and the like can be performed. The cell production management system 20 may be installed in the same facility as the health risk information management device 10, or in a different facility.

Based on instructions from the health risk information management device 10 and the like, the cell production management system 20 introduces an inducer into somatic cells collected from the user to produce iPS cells, and conducts quality tests (differentiation inducibility test, gene mutation test, fungus/bacteria/virus tests, and the like) of the produced iPS cells to produce standard-compliant iPS cells. The iPS cells thus produced are preserved under properly temperature-controlled conditions. Note that the cell production management system 20 can also produce specific somatic cells from iPS cells in addition to the production of the iPS cells. For example, the cell production management system 20 can produce iPS cells from skin tissue of the user and further produce various types of somatic cells from the produced iPS cells. For example, the somatic cells include nervous system cells, cardiac muscle, hepatocytes, β-cells, blood cells, platelets, skin cells, mesenchymal cells, etc. The conduction of demonstration experiments on health risks using somatic cells produced from iPS cells may also be managed in the cell production management system 20. Further, the cell production management system 20 may conduct verification experiments about compatibility between somatic cells produced from iPS cells of donor candidates and immune cells from the blood of a recipient based on instructions from the health risk information management device 10.

The user terminals 30 are terminals owned by users who have registered information in the health risk information management device 10. As the user terminals 30, all kinds of terminal devices capable of exchanging data with the health risk information management device 10 through a communication network, such as personal computers (PCs), laptop PCs, tablet terminals, smartphones, mobile phones, and personal digital assistants (PDAs), can be used. Each of the user terminals 30 includes a processor, input devices such a keyboard and a mouse, various operation buttons, and a touch panel, a display device such as a liquid crystal display, a communication interface to connect to the communication network, and memory resources such as disk drives or semiconductor memories (ROM, RAM, etc.).

### (Proposal Processing for Somatic Cell Production)

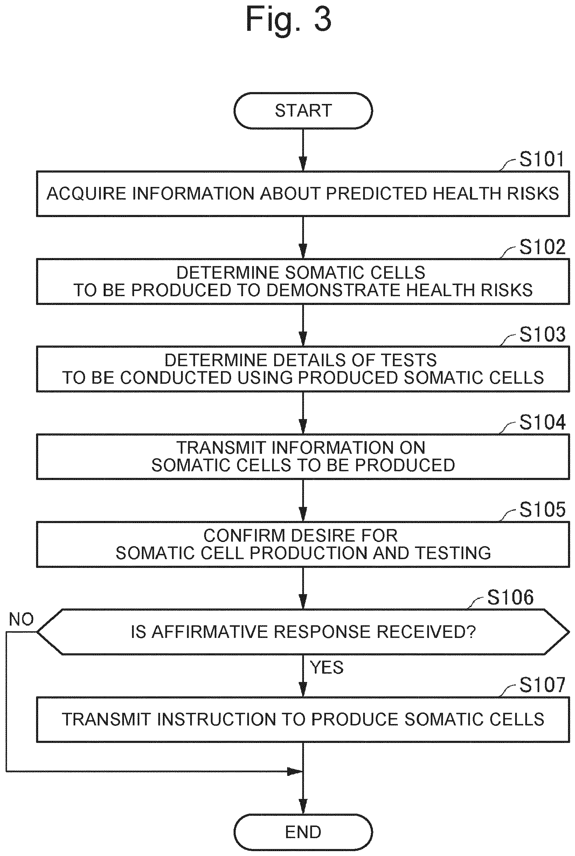

Next, proposal processing for somatic cell production based on health risk information on a user performed by the health risk information management device 10 will be described with reference to a flowchart of Fig. 3.

The health risk information management device 10 first acquires information about predicted health risks of the user (S101). The information about the predicted health risks is information estimated based, for example, on the results of genetic testing. The genetic testing includes testing using genome information such as whole exome sequencing (WES), whole genome sequencing (WGS), or SNP array, and testing using gene expression information, micro-RNA expression information, or test or sequencing for determining the HLA type or the like. The predicted health risks include information on diseases that a user (a person to be tested) and his/her family may develop in the future and the probability of developing each disease. The information on the predicted health risks may also be acquired through a communication line from a system of an institution or the like at which the genetic testing is conducted, or a technician operating the health risk information management device 10 or an administrator may refer to user's test data to enter the information.

Next, the health risk information management device 10 determines the type of somatic cells to be produced from cultured cells derived from the user in order to demonstrate the acquired predicted health risks of the user (S102). Here, the cultured cells are specifically iPS cells, but the cultured cells may also be any other type of stem cells, pluripotent stem cells, or somatic stem cells. For example, the cultured cells to be produced as the somatic cells are nerve cells when the user is expected to be at high risk of developing Alzheimer's disease, cartilage tissue cells when the user is expected to be at high risk of getting rheumatism, or cardiac muscle cells when the user is expected to be at high risk of getting a myocardial infarction. Further, the health risks are not limited to specific disease names. For example, the health risks also include information such as the fact that the UV resistance of the skin is weak and the skin is easy to dry when exposed to ultraviolet rays. In this case, skin cells are determined as the somatic cells to be produced.

Further, the health risk information management device 10 determines the details of tests to be conducted using the produced somatic cells in order to demonstrate the predicted health risks of the user (S103). For example, when the user is at high risk of developing Alzheimer's disease, an experiment is conducted in which the produced nerve cells are exposed to hydrogen peroxide or the like to check the vulnerability to oxidative stress. Further, when the user is at high risk of getting rheumatism, an experiment is conducted in which the produced cartilage tissue cells are used to check whether the symptoms of rheumatism appear or not. Further, when the user is expected to be at high risk of getting a myocardial infarction, an experiment is conducted in which the produced cardiac muscle cells are used to verify if the myocardial infarction actually develops or not. Further, when the UV resistance of the skin is weak, the produced skin cells are irradiated with UV to check the reaction or a dry test is performed to check the vulnerability to dryness. In addition, it is also effective for demonstrations when the user is at high risk of developing ALS (Amyotrophic Lateral Sclerosis), Parkinson's disease, skin disease, neurological disorder, eye disease, heart disease, liver disease, kidney disease, blood disease, and the like. Further, in addition to the demonstration of developing risks, responsiveness (allergic reaction, hypersensitivity, or the like) to existing drugs prescribed for these diseases, the presence or absence of allergies, toxicity, and the like may also be verified to verify which combination of drugs is most effective. Thus, the actually produced somatic cells are used to verify the likeliness of diseases or symptoms to appear, effective medical treatments, and preventive measures. The risk of GVHD (graft-versus-host disease) after transplant may also be verified.

Next, the health risk information management device 10 transmits, to the user terminal 30 of the user, information on somatic cells to be produced for conducting testing to verify the predicted health risks (S104), and accepts a response to whether or not the user desires to produce the somatic cells and conduct the testing (S105).

When receiving a response that the user desires to produce the somatic cells and conduct the testing (S106: YES), the health risk information management device 10 transmits, to the cell production management system 20, an instruction to produce the somatic cells (S107).

### (Demonstration Experiments on Health Risks Using Somatic Cells Produced Through iPS Cells, and Proposal Processing for Banking of iPS Cells Based on Demonstration Experiments)

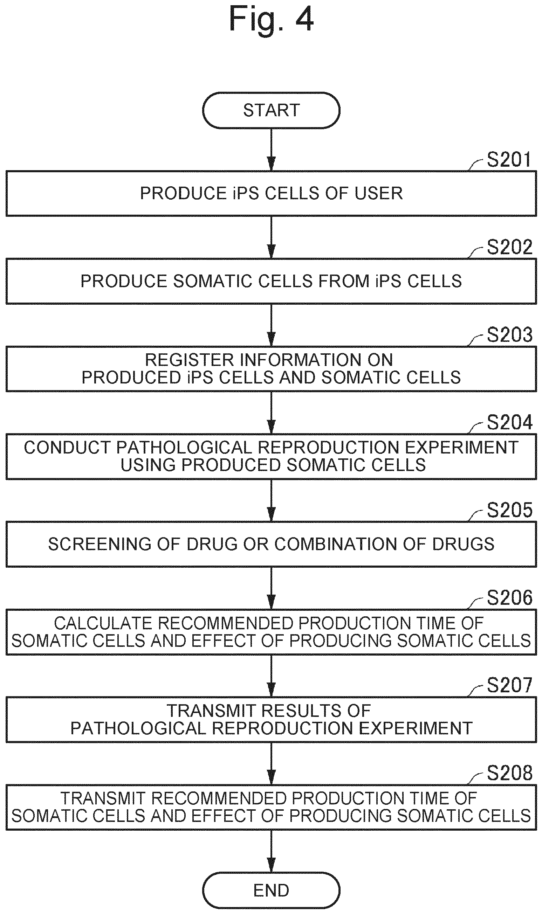

Next, demonstration experiments on health risks using somatic cells produced through iPS cells, and proposal processing for banking of iPS cells based on the demonstration experimental results will be described using a flowchart of Fig. 4. Note that respective steps of S201 to S205 illustrated in Fig. 4 are automatically performed by the cell production management system 20. Alternatively, a work flow may be automatically created by the cell production management system 20 so that an operator performs the respective steps based on operating instructions presented from the cell production management system 20.

In the cell production management system 20, iPS cells of the user is first produced (S201), and after that, somatic cells used to conduct the demonstration experiments of the health risks are produced through the iPS cells (S202). The produced iPS cells and somatic cells are managed with a tag (identification number). The tag information on the iPS cells and the somatic cells is also supplied to the health risk information management device 10 and registered in the customer information database 121 (S203).

Further, an experiment of a pathological reproduction model is carried out by using the somatic cells produced in the cell production management system 20 (S204). Specifically, an experiment to verify how likely a disease predicted to be at a risk to develop is conducted along the details determined in step S103. A period before the onset of the disease is also predicted. Further, the produced somatic cells are used to perform screening of a drug or a combination of drugs effective for improvement of pathology (S205). Specifically, it is verified what kind of drug is effective, what kind of drug combination is effective, how toxic each drug is, whether the user has an allergy to the drug, and the like. The experimental results using the pathological reproduction model (the probability of disease onset and a predicted period before the onset) and information on the drug screening results are registered in the customer information database 121.

Based on the experimental results using the pathological reproduction model, the health risk information management device 10 calculates a recommended time when iPS cells are produced in advance or when somatic cells are produced from the iPS cells, and the effect (probability) of eliminating health risks as a result of producing the somatic cells to prepare for a case where the disease actually develop in the future (S206).

The health risk information management device 10 transmits, to the user terminal 30 of the user, the results (the probability of disease onset and a predicted period before the onset) of the demonstration experiment conducted using the somatic cells (S207). In addition to the testing results, at least one of information on the somatic cells to be produced and preserved in case of future onset of the disease, the recommended production time, and numerical information on the effect of producing the somatic cells is also provided (S208). Further, based on the predicted period before the onset of the disease, the health risk information management device 10 provides at least one of information on required treatment costs and information on a payment plan (including a funded plan) of the treatment cost according to the period before the onset of the disease.

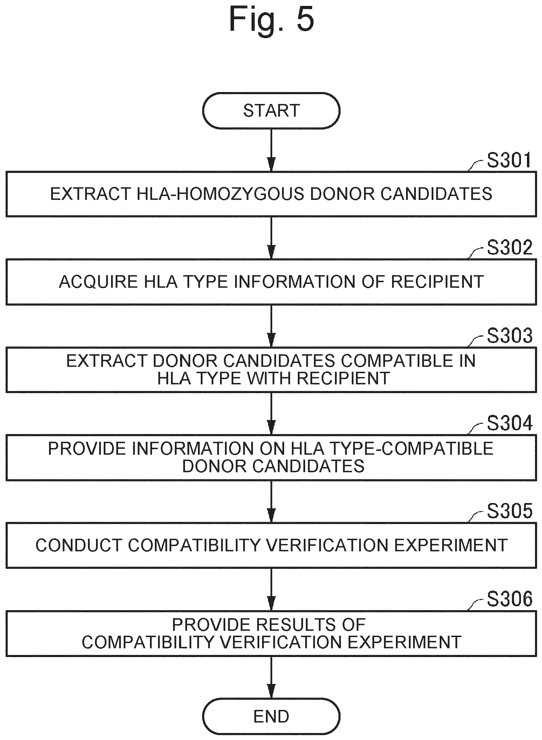

Next, HLA-homozygous donor search processing will be described using a flowchart of Fig. 5. In the embodiment, upon request from a person (hereinafter a recipient) who hopes the donation of somatic cells from donors, donors whose HLA type is compatible are searched for from users whose data are managed in the health risk information management device 10. The recipient may be a user whose data is managed in the health risk information management device 10, or an unregistered person. Further, the search for donors whose HLA type is compatible may be performed using any other database.

### (HLA-Homozygous Donor Search Processing)

First, the health risk information management device 10 extracts HLA-homozygous users from the users registered in the customer information database 121 (S301). The extracted users become donor candidates. In addition to the users registered in the customer information database 121, data managed at genetic testing companies, prenatal diagnosis data, data managed by a bone marrow bank, an HLA typing database, and the like may also be used to extract donor candidates from users with HLA information recorded thereon.

Next, the health risk information management device 10 acquires HLA type information of the recipient (S302) to extract, from the extracted donor candidates, donor candidates compatible with the recipient in terms of the HLA type (S303). Note that the extraction of donor candidates may also be done in such a manner that persons whose HLA types are similar to that of the recipient are extracted regardless of whether to be HLA homozygous or not.

The health risk information management device 10 may also provide information on HLA type-compatible donor candidates to the recipient (S304). Specifically, the information may be e-mailed to the user terminal 30 owned by the recipient, or may be printed out as a report.

After the donor candidates are determined, somatic cells produced from iPS cells of each of the donor candidates are mixed in vitro with immune cells derived from the blood of the recipient in a testing institute or the like to conduct a compatibility verification experiment (S305). The compatibility verification experiment may also be conducted by the cell production management system 20 based on an instruction from the health risk information management device 10. Alternatively, an operator may conduct the compatibility verification experiment based on work instructions presented from the health risk information management device 10. The results of the compatibility experiment may also be provided to the recipient through the health risk information management device 10 (S306). Further, information on the health risks of each donor (what kind of disease can develop and how likely the disease to develop) may be provided together with the results of the compatibility experiment.

Note that, based on data obtained by donor search processing upon request from many recipients, the health risk information management device 10 may also calculate, for each HLA homozygous user (each donor candidate), the number of recipients whose HLA type is compatible with the HLA homozygous user. Further, based on the number of recipients whose HLA type is compatible, the health risk information management device 10 may calculate, for each donor candidate, how many iPS cells of the donor candidate are required, and provide the calculation result to the cell production management system 20. Based on the calculation result, the cell production management system 20 can carry out the production and expanded culture of iPS cells of each donor candidate.

### (Determination of Production of Cultured Cells)

The health risk information management device 10 also has a function to determine, based on the acquired information on the predicted health risks of each user, whether or not to produce cultured cells derived from cells (including somatic cells, stem cells, mesenchymal stem cells, blood cells, epithelial cells, and the like) of the user. Like in the aforementioned embodiment, the cultured cells are specifically iPS cells, but the cultured cells may also be any other type of stem cells, pluripotent stem cells, or somatic stem cells. Thus, the determination of whether or not to produce and preserve iPS cells can be made precisely according to the potential for and severity of the future health risks of the user. As an example in which the preparation of iPS cells is determined to be effective, for example, there is a case where effective medical treatment can be performed by using the iPS cells when diseases actually develop. Further, in case of diseases with a strong tendency to be inherited across blood relatives, the determination result that the stock of iPS cells is effective may also be given to other persons as the blood relatives if the blood relatives such as parents and brothers are at risk of these diseases. Note that the production of cells may also include cell initialization, reprogramming, cell-fate conversion, and cell transformation.

The health risk information management device 10 further has a function to determine whether or not to produce cells derived from user's somatic cells based on the information on the HLA type of each user. Specifically, since it is possible for an HLA homozygous user to become a donor, the determination that the creation and preservation of iPS cells are effective may also be made.

As described above, appropriate advice can be given to users efficiently by determining whether each user is a user to which the production and preservation of iPS cells should be recommended or not on the basis of predicted health risks based on genetic testing and information on the HLA type. Especially, since the production and preservation of iPS cells are expensive, advice based on accurate information is important. Further, since the production of iPS cells can be prioritized for those who really need them, the resources such as the cell production management system 20 can also be used effectively.

As described above, according to the embodiment, there can be provided a scheme to determine the type of somatic cells to be produced from iPS cells derived from somatic cells of a user and to conduct tests for demonstrating predicted health risks using the produced somatic cells in order to demonstrate the predicted health risks of the user based on genome information and the like. This can make use of iPS cells to predict future health risks accurately.

Further, a scheme is realized in which, after information on somatic cells required for testing is provided to each user, a response to whether or not the user desires to produce and test iPS cells and somatic cells derived from the iPS cells is accepted from the user, and when the affirmative response is accepted, the production of somatic cells is instructed to the cell production management system 20. Thus, the production and testing of iPS cells can be managed efficiently according to the user's desire.

Further, information on somatic cells to be produced and preserved for the user based on the results of testing using the somatic cells in case of future onset of diseases, the recommended production time, and numerical information on the effect of producing the somatic cells are provided. Thus, the user can get information for making a decision about appropriate preparation for future health risks. Specifically, the information becomes criteria for the user to make a decision on whether or not to produce and preserve the iPS cells of the user for the future.

Further, since a drug or a combination of drugs effective for improvement of the predicted health risks is verified by using the produced somatic cells, and the information is provided to the user, the somatic cells produced from the iPS cells can be used effectively.

Further, a scheme is realized in which HLA homozygous users are extracted as donor candidates to extract, from the donor candidates, donor candidates whose HLA type is compatible with a recipient who hopes the donation of somatic cells from donors. Thus, the donors having the compatible HLA type can be found efficiently. Further, since a scheme is realized in which compatibility is verified by using immune cells derived from the blood of the recipient and somatic cells derived from iPS cells of each of the donor candidates having the compatible HLA type to provide the verification results, the recipient can receive the somatic cells from the donor after immune rejection reaction is verified in advance. Note that the extraction of donor candidates may also be done in a manner to extract people similar in HLA type to the recipient regardless of whether to be HLA homozygous or not. For example, there are 15 HLA types, and it can be generally treated with a weak immunosuppressant if four of those HLA types are compatible. Then, as an example, it is considered that no immunosuppressant is unnecessary as long as six of those HLA types are compatible. Therefore, donor candidates may also be extracted based on information on how many HLA types are compatible. Note that the criteria to determine whether the HLA type is similar or not are not limited thereto.

Further, based on the HLA type of the user, iPS cells the HLA type of which matches or is similar may be extracted from iPS cells of people stored in the cell production management system 20. Further, cells derived from the selected HLA type may be used to verify whether the cells have the effect of suppressing GVHD or not.

As for HLA homozygous users (donor candidates), the number of recipients whose HLA type is compatible may also be managed. Further, based on the number of recipients whose HLA type is compatible, how many cultured cells derived from cells of each of donor candidates are needed may be calculated to produce iPS cells of each donor candidate based on the calculation result. This can produce iPS cells and somatic cells systematically so that each recipient can receive somatic cells from each donor whose HLA type is compatible.

Note that the prediction accuracies of various prediction processing such as health risks and HLA compatibility performed by the health risk information management device 10 of the present invention can be all improved by using deep learning or AI (Artificial Intelligence).

Note that the present invention is not limited to the embodiment described above, and the present invention can be carried out in various other forms without departing from the scope of the present invention. Therefore, the aforementioned embodiment is just an example in all respects and should not be interpreted in a limited manner. For example, respective processing steps described above can be executed by changing the order arbitrarily or executed in parallel as long as there is no contradiction in the processing contents.

### Reference Signs List

- 10: health risk information management device
- 11: control device
- 12: external storage device
- 20: cell production management system
- 30: user terminal
- 101: predicted health risk information acquisition unit
- 102: somatic cell-type determination unit
- 103: recommended test detail determination unit
- 104: somatic cell production instruction unit
- 105: test result providing unit
- 106: cell production necessity confirmation unit
- 107: health risk treatment information providing unit
- 108: donor candidate extraction unit
- 109: recipient information acquisition unit
- 110: HLA compatibility determination unit
- 111: compatibility information providing unit
- 112: number-of-compatible person calculation unit
- 113: donor cell production management unit
- 114: cultured cell production determination unit
- 121: customer information database

## Claims

1. A health risk information management device comprising:
a predicted health risk information acquisition unit which acquires information about predicted health risks of a user; and
a cultured cell production determination unit which determines whether or not to produce cultured cells derived from the user based on the information on the predicted health risks.

2. The health risk information management device according to claim 1, wherein the predicted health risk information acquisition unit acquires information about predicted health risks based on information on genetic testing of the user.

3. The health risk information management device according to claim 1 or 2, further comprising a somatic cell-type determination unit which determines a type of somatic cells to be produced from cultured cells derived from the user based on the information on the predicted health risks.

4. The health risk information management device according to claim 3, further comprising a recommended test detail determination unit which determines details of tests to be performed using somatic cells produced from cultured cells derived from the user based on the information on the predicted health risks.

5. The health risk information management device according to claim 4, further comprising:
a somatic cell production instruction unit which transmits a somatic cell production instruction to a system which manages the cell production; and
a test result providing unit which provides, to the user, results of the tests conducted using produced somatic cells.

6. The health risk information management device according to claim 5, further comprising
a cell production necessity confirmation unit which provides, to the user, information of the somatic cells to be produced, and accepts a response to whether production of somatic cells is desired or not,
wherein when an affirmative response that the production of somatic cells is desired is accepted, the somatic cell production instruction unit transmits a production instruction of the somatic cells to the system which manages the production of the somatic cells.

7. The health risk information management device according to claim 6, wherein the cell production necessity confirmation unit accepts a response to whether production of the cultured cells is desired or not.

8. The health risk information management device according to claim 5, further comprising a health risk treatment information providing unit which provides information about preventive measures against the predicted health risks of the user based on the test results and countermeasures when predicted health risks occur.

9. The health risk information management device according to claim 8, wherein the health risk treatment information providing unit provides at least either information of a drug screened by using the produced somatic cells to be effective for improvement of the predicted health risks, and information on drug toxicity and responsiveness to the drag.

10. The health risk information management device according to claim 8, wherein the health risk treatment information providing unit provides information on type of somatic cells to be produced from cultured cells derived from the user based on the test results.

11. The health risk information management device according to claim 8, wherein the health risk treatment information providing unit provides information of somatic cells to be produced and preserved by the user for the future based on the test results, a recommended production time, and numerical information on an effect of producing the somatic cells.

12. The health risk information management device according to claim 8, wherein the health risk treatment information providing unit provides information about the cultured cells to be produced and preserved by the user based on the test results.

13. The health risk information management device according to claim 8, wherein the health risk treatment information providing unit predicts a period before occurrence of the health risks based on the test results, and provides information on required treatment costs and a payment plan according to the period before the occurrence.

14. The health risk information management device according to claim 1, further comprising
a donor candidate extraction unit which extracts HLA homozygous users as donor candidates based on information on HLA types of users,
wherein based on whether a user is HLA homozygous or not, the cultured cell production determination unit determines whether to produce cultured cells derived from the user or not.

15. The health risk information management device according to claim 14, further comprising:
a recipient information acquisition unit which acquires information on an HLA type of a recipient who hopes donation of somatic cells from a donor whose HLA type is compatible; and
an HLA compatibility determination unit which extracts donor candidates whose HLA type is compatible with the recipient from among extracted HLA-homozygous donor candidates.

16. The health risk information management device according to claim 15, further comprising a compatibility information providing unit which provides, to the recipient, results of compatibility verification performed by using immune cells derived from blood of the recipient and iPS cells of donor candidates whose HLA type is compatible with the recipient or somatic cells derived from the iPS cells.

17. The health risk information management device according to claim 14, further comprising a number-of-compatible person calculation unit which calculates the number of recipients whose HLA type is compatible with each of donor candidates.

18. The health risk information management device according to claim 14, further comprising a donor cell production management unit which calculates how many cultured cells derived from each of donor candidates are needed based on the number of recipients whose HLA type is compatible.

19. The health risk information management device according to claim 15, wherein the HLA compatibility determination unit extracts cultured cells having compatible HLA type from among preserved cultured cells based on information on HLA types of users.

20. The health risk information management device according to claim 15, wherein when a predetermined number of HLA types is compatible among a plurality of HLA types, the HLA compatibility determination unit determined to be HLA-type compatible.

21. The health risk information management device according to any one of claims 1 to 20, wherein cultured cells derived from the cells are stem cells.

22. A health risk information management method comprising:
a step of acquiring information about predicted health risks of a user; and
a step of determining whether or not to produce cultured cells derived from the user based on the information on the predicted health risks.

23. A program causing a computer to function as:
a predicted health risk information acquisition unit which acquires information about predicted health risks of a user; and
a cultured cell production determination unit which determines whether or not to produce cultured cells derived from the user based on the information on the predicted health risks.
